# EUROPÄISCHE PATENTSCHRIFT

(11) **EP 4 400 146 B1**
(45) Veröffentlichungstag und Bekanntmachung des Hinweises auf die Patenterteilung: **19.11.2025**
(21) Anmeldenummer: 24178290.3
(22) Anmeldetag: 27.02.2019
(51) Int. Cl.: A61M 16/00, A61M 16/08, A61M 16/12, A61M 16/20, A61M 16/10

(54) **BEATMUNGSGERÄT MIT SCHALTVENTIL**
RESPIRATORY APPARATUS WITH SWITCHING VALVE
APPAREIL RESPIRATOIRE À SOUPAPE DE COMMANDE

(30) Priorität: 08.03.2018 DE 102018001888
(43) Veröffentlichungstag der Anmeldung: 17.07.2024
(62) Teilanmeldung aus: 19159730.1
(73) Patentinhaber: Löwenstein Medical Technology S.A., 2557 Luxembourg (LU)
(72) Erfinder: Adametz, Benjamin, 22609 Hamburg (DE); Göbel, Christof, 22457 Hamburg (DE)
(74) Vertreter: Löwenstein Medical IP

(56) Entgegenhaltungen:
- WO-A1-2017/029629
- WO-A2-2012/085792
- CN-A- 105 617 527
- CN-A- 107 308 531
- GB-A- 2 101 895
- US-A- 4 003 377
- US-A1- 2008 178 882
- US-A1- 2012 145 156
- US-B2- 8 893 718

## Beschreibung

Die vorliegende Erfindung betrifft ein Beatmungsgerät mit einem Geräteeingang und einem Geräteausgang und einem Atemgasweg zwischen Geräteeingang und Geräteausgang, wobei im Atemgasweg ein Atemgasantrieb, ein Rückschlagventil und ein Schaltventil angeordnet sind.

Beatmungsgeräte werden für die Therapie respiratorischer Störungen eingesetzt, dabei können die Beatmungsgeräte in der nicht- invasiven und invasiven Beatmung, sowohl innerklinisch als auch außerklinisch, verwendet werden.

Bei einer Beatmung eines Patienten kann in der Regel ein Beatmungsgerät mit einem inspiratorischen Zweig für den Atemgasfluss und optional mit einem Zweig für den exspiratorischen Atemgasfluss eingesetzt werden. Der Zweig für den exspiratorischen Atemgasfluss ermöglicht die Ausatmung/Exspiration eines Atemgases durch den Patienten, während der Zweig für den inspiratorischen Atemgasfluss den Patienten mit Atemgas versorgt.

GB 2 101 895 offenbart ein Beatmungsgerät umfassend einen inspiratorischen und einen exspiratorischen Atemgasweg, einen Geräteeingang und einen Geräteausgang, wobei im inspiratorischen Atemgasweg ein Atemgasantrieb, ein Rückschlagventil, zwei Überdruckbegrenzungsventile und ein manuelles Schaltventil angeordnet ist. Das Rückschlagventil ist eingerichtet, einen Atemgasstrom in einer Richtung vom Geräteausgang zum Geräteeingang zu verhindern. Das manuelle Schaltventil ist in einem Bypass zu dem Rückschlagventil angeordnet, wodurch das Rückschlagventil durch Öffnen des manuellen Schaltventils umgangen und ein Gas vom Geräteausgang Richtung Geräteeingang rückgeführt werden kann.

CN 107 308 531 offenbart ein Ventil mit einem Rückschlagventil und ein Beatmungsgerat mit einem solchen Ventil. Das Ventil regelt den inspiratorischen Druck. Zudem ist das Ventil eingerichtet, eine exspiratorische Rückatmung des Patienten in das Beatmungsgerat zu verhindern.

CN 105 617 527 offenbart ein Ausatemventil zur Verwendung mit einem Beatmungsgerat mit einem Bypass zur Umgebungsluft. Der Bypass öffnet bei einem Ausfall des Beatmungsgeräts und ermöglicht dem Patienten eine eigenständige Atmung. Die Aufgabe ist, dass Ausatemluft in die Umgebung abgeleitet wird und somit nicht in das Beatmungsgerat zurück geatmet wird.

US 2012/145156 offenbart ein Sprechventil zur Verwendung bei beatmeten Patienten, die ein Tracheostoma haben und daher unter der Beatmung nicht sprechen können.

US 8 893 718 offenbart ein Anästhesiegerät mit einer Beatmungseinheit. Die Beatmungseinheit weist einen inspiratorischen und einen exspiratorischen Zweig auf. Beide Zweige sind unidirektional. Im Bereich einer patientennahen Zweigstelle ist ein Druckentlastungsventil angeordnet.

Bei den im Stand der Technik bekannten Beatmungsgeräten kann es zu sowohl zu Blockaden/Störungen im exspiratorischen Zweig als auch im inspiratorischen Zweig des Beatmungsgerätes kommen.

Eine Blockade/Störung im exspiratorischen Zweig kann beispielsweise durch Materialeintrag, wie beispielsweise Medikamente oder Sekret, verursacht werden. Blockaden/Störungen im exspiratorischen Zweig können einen Patienten an einer Ausatmung von Atemgas hindern.

Eine Blockade/Störung des exspiratorischen Zweiges kann beispielsweise dazu führen, dass der Atemwegsdruck in der Exspiration nicht auf das angestrebte Druckniveau gesenkt werden kann, wodurch ein Patient nicht ausatmen kann. Ein anhaltender erhöhter Atemgasdruck in Atemwegen des Patienten kann schädliche Folgen haben. Beispielsweise kann es zu einem gefährlichen Anstieg des intrathorakalen Drucks kommen, der zu einem verminderten venösen Rückfluss, verminderter Herzleistung und schließlich zu einem Abfall des arteriellen Blutdrucks führt.

Neben der Problematik einer Blockade/Störung im exspiratorischen Zweig des Beatmungsgerätes kann es auch zu einer Blockade/Störung im inspiratorischen Zweig des Beatmungsgerätes kommen. Eine Blockade/Störung im inspiratorischen Zweig kann beispielsweise durch einen Ausfall des Atemgasantriebs hervorgerufen werden. Blockaden/Störungen im inspiratorischen Zweig können Patienten an einer Einatmung hindern oder diese zumindest erschweren.

Die im Stand der Technik bekannten Beatmungsgeräte weisen somit den Nachteil auf, dass sie auch bei vorhandenen Sicherungsvorrichtungen nicht ausreichend gegen Blockaden, Störungen oder Ausfälle gesichert sind, sodass eine Inspiration und/oder Exspiration von Atemgas nicht sichergestellt werden kann.

Es ist daher Aufgabe der vorliegenden Erfindung, ein Beatmungsgerät zur Verfügung zu stellen, das eine Sicherung einer Inspiration und/oder einer Exspiration von Atemgas bei einer Störung / Blockade eines inspiratorischen oder exspiratorischen Zweiges des Beatmungsgerätes sicherstellt.

Der Gegenstand der Erfindung wird durch den unabhängigen Anspruch definiert. Die Unteransprüche definieren Ausführungsbeispiele der Erfindung.

Hierin beschrieben wird ein Beatmungsgerät mit mindestens einem Geräteeingang und einem Geräteausgang und einem Atemgasweg zwischen Geräteeingang und Geräteausgang, wobei im Atemgasweg ein Atemgasantrieb, ein Rückschlagventil und ein Schaltventil angeordnet sind. Der Atemgasweg ist ein inspiratorischer Atemgasweg. Das Beatmungsgerä umfasst mindestens einen inspiratorischen Atemgasweg und mindestens einen exspiratorischen Atemgasweg. Mindestens ein Rückschlagventil ist in mindestens einem inspiratorischen Atemgasweg angeordnet.

Das Rückschlagventil kann zwischen dem Geräteeingang und dem Atemgasantrieb angeordnet sein. Alternativ kann das Rückschlagventil zwischen dem Atemgasantrieb und dem Geräteausgang angeordnet sein. Ein Atemgasantrieb kann ein Gebläse oder eine Druckquelle, insbesondere eine Sauerstoff- oder Luftdruckquelle, sein.

Das Rückschlagventil verhindert einen Atemgasstrom in einer Richtung vom Geräteausgang zum Geräteeingang und das Schaltventil ermöglicht zumindest zeitweise einen Atemgasstrom in einer Richtung vom Geräteausgang zum Geräteeingang oder zu einer separaten Geräteöffnung. Die separate Geräteöffnung kann dabei ein zweiter/separater Geräteeingang oder ein zweiter/separater Geräteausgang sein. Ein Schaltventil kann elektromechanisch ausgebildet sein. Beispielsweise kann ein Schaltventil ein Pneumatikventil mit einem Magentventil sein bzw. ein Bypass mit einem Magnetventil. Ein Schaltventil kann ein 3/2-Wegeventil, ein Pneumatikventil oder ein Membranventil sein.

Ausgeatmetes Atemgas des Patienten kann über einen exspiratorischen Atemgasweg des Beatmungsgerätes abgeführt werden. Eine Rückführung des Atemgases über den inspiratorischen Atemgasweg kann somit verhindert werden.

Das Rückschlagventil verhindert eine ungewollte Kontamination des Atemgasantriebs mit verunreinigtem Atemgas des Patienten bei einem normalen Betrieb des Beatmungsgerätes. Durch das umfasste Schaltventil kann, bei einer Blockade/Störung des exspiratorischen Atemgaswegs, zumindest zeitweise das Rückschlagventil umgangen werden. Dies bietet den Vorteil, dass bei einer Blockade des exspiratorischen Atemgasweges Atemgas des Patienten über den inspiratorischen Atemgasweg abgeführt werden kann.

Zwischen dem Rückschlagventil und dem Geräteausgang ist eine Flussmesseinrichtung angeordnet. Die Flussmesseinrichtung ist eingerichtet ein Volumen des Atemgases im Atemgasweg zu erfassen. Das Schaltventil ist eingerichtet, basierend auf dem erfassten Volumen des Atemgases öffnenbar oder schließbar zu sein. Dies ermöglicht eine atemgasvolumen- oder atemgasdruckabhängige Schaltung des Schaltventils. Das Schaltventil kann beispielsweise durch ein 3/2-Wegeventil schaltbar sein.

Das Schaltventil ist in einem Bypass zu dem Rückschlagventil angeordnet. Der Bypass kann offen oder geschlossen ausgebildet sein. Geschlossen bedeutet, dass der Bypass in den Atemgasweg einmündet, von dem er abgezweigt ist und dabei das Rückschlagventil umgeht. Das Schaltventil ist in dem Bypass angeordnet. Offen bedeutet, dass der Bypass nicht in den Atemgasweg einmündet, von dem er abgezweigt ist. Vielmehr ist ein offener Bypass ein Atemgasweg, der beispielsweise über einen separaten/zweiten Geräteausgang verfügen kann. Der offene Bypass zweigt in der Regel zwischen dem Gerätausgang und dem Atemgasantrieb oder dem Rückschlagventil ab und mündet in dem separaten/zweiten Geräteausgang. Alternativ kann der offene Bypass zwischen dem Rückschlagventil und dem Atemgasantrieb abzweigen und in den zweiten/separaten Geräteausgang münden. In dem offenen Bypass kann eine Flussmesseinrichtung angeordnet sein. In der Regel ist die Flussmesseinrichtung zwischen dem zweiten/separaten Geräteausgang und dem Bypass angeordnet. In dem offenen Bypass kann ein separates Schaltventil angeordnet sein. Das Schaltventil kann zeitgesteuert, patientengetriggert oder basierend auf einem durch die Flussmesseinrichtung erfassten Fluss bzw. Volumen oder Druck des Atemgases schaltbar sein. In der Regel ist die Flussmesseinrichtung zwischen dem Schaltventil und dem gemeinsamen Geräteausgang angeordnet. Optional kann der offene Bypass zusätzlich zu einem geschlossenen Bypass ausgebildet sein. Der offene Bypass bietet den Vorteil, dass von dem Patienten abgegebenes Atemgas über den separaten/zweiten Geräteausgang an die Umgebung abgegeben werden kann.

In einer Weiterbildung bildet das mindestens eine Schaltventil einen Bypass um das Rückschlagventil aus. Durch die Ausbildung des geschlossenen Bypasses um das Rückschlagventil, kann das Rückschlagventil derart umgangen werden, dass das Atemgas um das Rückschlagventil herum in den selben Atemgasweg eingeleitet wird, von dem es abgezweigt wurde. Optional bildet das mindestens eine Schaltventil einen offenen Bypass um das Rückschlagventil aus. Bei dem offen ausgebildeten Bypass kann Atemgas bei einer Blockade/Störung des inspiratorischen Atemgaswegs über den offenen Bypass an dem Rückschlagventil vorbei über einen separaten Geräteausgang abgeführt werden.

In Ausgestaltung ist das mindestens eine Schaltventil eingerichtet, den Bypass zu öffnen und/oder zu schließen, wobei das Schaltventil automatisch schaltbar eingerichtet ist. Das Schaltventil ist eingerichtet, basierend auf dem ermittelten Volumen des Atemgases automatisch zu öffnen oder zu schließen.

In Ausgestaltung ist der Atemgasantrieb zwischen dem Geräteeingang und dem Rückschlagventil angeordnet und eingerichtet, Atemgas in Richtung des Rückschlagventils und des Geräteausgangs zu fördern. Die Anordnung des Atemgasantriebs definiert eine Saug- und eine Druckseite im Beatmungsgerät. Die Saugseite ist die Seite in Atemgasflussrichtung vor dem Atemgasantrieb. Die Druckseite ist die Seite in Atemgasflussrichtung dem Atemgasantrieb nachgeschaltet. Durch die Anordnung des Atemgasantriebs direkt hinter dem Geräteeingang befinden sich das Rückschlagventil, das Schaltventil und die Flussmesseinrichtung auf der Druckseite des Beatmungsgerätes.

In einer Ausgestaltung zweigt der Bypass zu dem Schaltventil zwischen dem Atemgasantrieb und dem Rückschlagventil ab und mündet zwischen dem Rückschlagventil und dem Geräteausgang wieder in den Atemgasweg. Durch diese Anordnung wird der Atemgasantrieb bei einem normalen Betrieb des Beatmungsgerätes vor einer ungewollten Kontamination durch rückgeführtes Atemgas geschützt.

In einer alternativen Ausgestaltung ist der Atemgasantrieb zwischen dem Rückschlagventil und dem Geräteausgang angeordnet und eingerichtet, Atemgas in Richtung des Geräteausgangs zu fördern. Durch die Anordnung des Atemgasantriebs zwischen dem Rückschlagventil und dem Geräteausgang befinden sich das Rückschlagventil und das Schaltventil auf der Saugseite des Beatmungsgerätes.

In einer Weiterbildung der alternativen Ausgestaltung zweigt der Bypass zu dem Schaltventil zwischen dem Geräteeingang und dem Rückschlagventil ab und mündet zwischen dem Rückschlagventil und dem Atemgasantrieb wieder in den Atemgasweg. Dies bietet den Vorteil, dass das Rückschlagventil umgangen werden kann. Zudem sind bei einer derartigen Anordnung das Schaltventil und das Rückschlagventil auf der Saugseite des Beatmungsgerätes angeordnet.

In einer weiteren Ausgestaltung ist zusätzlich zu dem Bypass ist ein zweiter Atemgasweg ausgebildet, der einen zweiten Geräteeingang und mindestens ein Rückschlagventil umfasst und vor dem gemeinsamen Geräteausgang in den ersten Atemgasweg mündet. Typischerweise ist der zweite Atemgasweg ein separater inspiratorischer Atemgasweg, der eingerichtet ist, bei einer Blockade/Störung des inspiratorischen Atemgaswegs einen separaten Atemgasweg bereitzustellen, über den Atemgas inspirierbar ist. Der zweite inspiratorische Atemgasweg kann einen separaten Atemgasantrieb umfassen. Beispielsweise ist der Atemgasantrieb zwischen dem zweiten Geräteeingang und dem Rückschlagventil des zweiten Atemgaswegs angeordnet. Ferner kann der zweite Atemgasweg eine separate Flussmesseinrichtung umfassen, die eingerichtet ist, den Fluss bzw. das Volumen oder den Druck des Atemgases im zweiten Atemgasweg zu erfassen.

In einer weiteren Ausgestaltung ist ein Atemgasweg, für einen exspiratorischen Atemgasfluss umfasst, der einen exspiratorischen Geräteeingang und einen exspiratorischen Geräteausgang umfasst. Der Atemgasweg für einen exspiratorischen Atemgasfluss ist eingerichtet, Atemgas des Patienten abzuführen. Der exspiratorische Geräteeingang ist dabei in der Regel patientennah angeordnet, während der exspiratorische Geräteausgang patientenfern ausgebildet ist. Der Atemgasweg für exspiratorisches Atemgas ist eingerichtet, Atemgas des Patienten an den exspiratorischen Geräteausgang abzuführen und somit dem Patienten ein Ausatmen zu ermöglichen.

In einer Weiterbildung führt von dem exspiratorischen Geräteeingang der Atemgasweg zu dem exspiratorischen Geräteausgang und in dem Atemgasweg sind ein Schaltventil und eine Flussmesseinrichtung angeordnet. Die Flussmesseinrichtung ist in der Regel zwischen dem exspiratorischen Geräteausgang und dem Schaltventil angeordnet. Das Schaltventil ist in der Regel eingerichtet, basierend auf einem festgelegten Zeitintervall oder einem Patiententrigger schaltbar zu sein. Alternativ kann das Schaltventil basierend auf einem durch eine Flussmesseinrichtung gemessenen Fluss bzw. Volumen oder Druck des Atemgases schaltbar sein. Die zwischen dem exspiratorischen Geräteausgang und dem Schaltventil angeordnete Flussmesseinrichtung ist typischerweise eingerichtet, den Fluss bzw. das Volumen oder den Druck des Atemgases zu erfassen und eine Rückmeldung über das durch den Patienten abgegebene Atemgas an das Beatmungsgerät zu ermöglichen.

In einer weiteren Weiterbildung führt zwischen dem exspiratorischen Geräteeingang und dem Schaltventil ein separater exspiratorischer Atemgasweg mit einem Schaltventil und einer Flussmesseinrichtung zu einem separaten Geräteausgang. Der separate exspiratorische Atemgasweg ist eingerichtet, Atemgas des Patienten beispielsweise bei einer Blockade/Störung des exspiratorischen Atemgaswegs abzuführen. Die zwischen dem exspiratorischen Geräteausgang und dem Schaltventil angeordnete Flussmesseinrichtung ist typischerweise eingerichtet, den Fluss bzw. das Volumen oder den Druck des Atemgases zu erfassen und eine Rückmeldung über das durch den Patienten abgegebene Atemgas zu ermöglichen.

In Ausgestaltung ist an den Geräteausgang ein Schlauchsystem adaptiert, das mit einem ersten Zweig zu einem Patienteninterface führt und vor dem Patienteninterface mit einem zweiten Zweig zu dem exspiratorischen Geräteeingang für exspiratorisches Atemgas führt. Optional ist das Schlauchsystem ein Einschlauchsystem. Alternativ kann an den Geräteausgang ein Schlauchsystem adaptiert sein, dass ein Leckageschlauchsystem ist. Durch das Schlauchsystem wird das Patienteninterface mit mindestens einem inspiratorischen Atemgasweg verbunden. Optional wird das Patienteninterface durch das Schlauchsystem mit mindestens einem inspiratorischen Atemgasweg und mindestens einem exspiratorischen Atemgasweg verbunden.

In einer Weiterbildung ist das Schaltventil in dem Bypass angeordnet wobei der Bypass als offener Bypass ausgebildet ist, der einen separaten Geräteausgang umfasst, wobei der offene Bypass von dem inspiratorischen Atemgasweg zwischen dem Rückschlagventil und dem Geräteausgang abzweigt und in den separaten Geräteausgang mündet, wobei der inspiratorische Atemgasweg zumindest ein weiteres Ventil und eine Flussmesseinrichtung aufweist, wobei die Flussmesseinrichtung zwischen dem Atemgasantrieb und dem Rückschlagventil angeordnet ist.

Hierin beschrieben wird zudem ein Beatmungsgerät mit mindestens einem Geräteeingang und einem Geräteausgang und einem Atemgasweg zwischen Geräteeingang und Geräteausgang, wobei im Atemgasweg ein Atemgasantrieb, ein Rückschlagventil und ein Schaltventil angeordnet sind.

Das Schaltventil ist in dem Bypass angeordnet, wobei der Bypass als offener Bypass ausgebildet ist, der einen separaten Geräteausgang umfasst, wobei der offene Bypass von dem inspiratorischen Atemgasweg zwischen dem Rückschlagventil und dem Geräteausgang abzweigt und in den separaten Geräteausgang mündet, wobei der inspiratorische Atemgasweg zumindest ein weiteres Ventil und eine Flussmesseinrichtung aufweist, wobei die Flussmesseinrichtung zwischen dem Atemgasantrieb und dem Rückschlagventil angeordnet ist.

Im Folgenden werden anhand von stark vereinfachten schematischen Darstellungen bevorzugte Ausführungsbeispiele näher erläutert. Es zeigt
- Fig. 1: einen schematischen Aufbau einer Ausführungsform eines Beatmungsgerätes mit einem inspiratorischen Atemgasweg mit einem Bypass um ein Rückschlagventil,
- Fig. 2: eine alternative Anordnung der in Fig. 1 gezeigten Ausführungsform des Beatmungsgerätes mit dem inspiratorischen Atemgasweg mit dem Bypass um ein Rückschlagventil,
- Fig. 3: einen schematischen Aufbau einer weiteren Ausführungsform des in Fig. 1 gezeigten Beatmungsgerätes, mit dem inspiratorischen Atemgasweg mit dem Bypass und einem zweiten inspiratorischen Atemgasweg,
- Fig. 4: eine alternative Anordnung der in Fig. 2 gezeigten Ausführungsform des in Fig. 1 gezeigten Beatmungsgerätes, mit dem inspiratorischen Atemgasweg mit dem Bypass und dem zweiten inspiratorischen Atemgasweg,
- Fig. 5: eine alternative Anordnung der in Fig. 1 und Fig. 3 gezeigten Ausführungsform des Beatmungsgerätes, mit dem inspiratorischen Atemgasweg mit dem Bypass und dem zweiten inspiratorischen Atemgasweg,
- Fig. 6: eine alternative Anordnung der in Fig. 2 und Fig. 4 gezeigten Ausführungsform des Beatmungsgerätes, mit dem inspiratorischen Atemgasweg mit dem Bypass und dem zweiten inspiratorischen Atemgasweg,
- Fig. 7: einen schematischen Aufbau einer erfindungsgemäßen Ausführungsform des Beatmungsgerätes mit dem inspiratorischen Atemgasweg mit dem Bypass und dem exspiratorischen Atemgasweg,
- Fig. 8: einen schematischen Aufbau einer weiteren Ausführungsform des Beatmungsgerätes mit dem inspiratorischen Atemgasweg und dem zweiten inspiratorischen Atemgasweg sowie mit einem exspiratorischen Atemgasweg und einem separaten exspiratorischen Atemgasweg,
- Fig. 9: einen schematischen Aufbau einer alternativen Ausführungsform des Beatmungsgerätes mit dem inspiratorischen Atemgasweg mit einem offenen Bypass mit einem separaten Geräteausgang,
- Fig. 10: einen schematischen Aufbau einer alternativen Ausführungsform des in Fig. 9 gezeigten Beatmungsgerätes mit dem inspiratorischen Atemgasweg mit dem offenen Bypass mit dem separaten Geräteausgang,
- Fig. 11: einen schematischen Aufbau einer alternativen Ausführungsform des in Fig. 9 gezeigten Beatmungsgerätes mit dem inspiratorischen Atemgasweg mit dem offenen Bypass mit dem separaten Geräteausgang,
- Fig. 12: einen schematischen Aufbau einer alternativen Ausführungsform des - in den Figuren 9 und 11 gezeigten-Beatmungsgerätes mit dem inspiratorischen Atemgasweg mit einem offenen Bypass mit einem separaten Geräteausgang.

In den Figuren weisen dieselben konstruktiven Elemente jeweils dieselben Bezugsziffern auf.

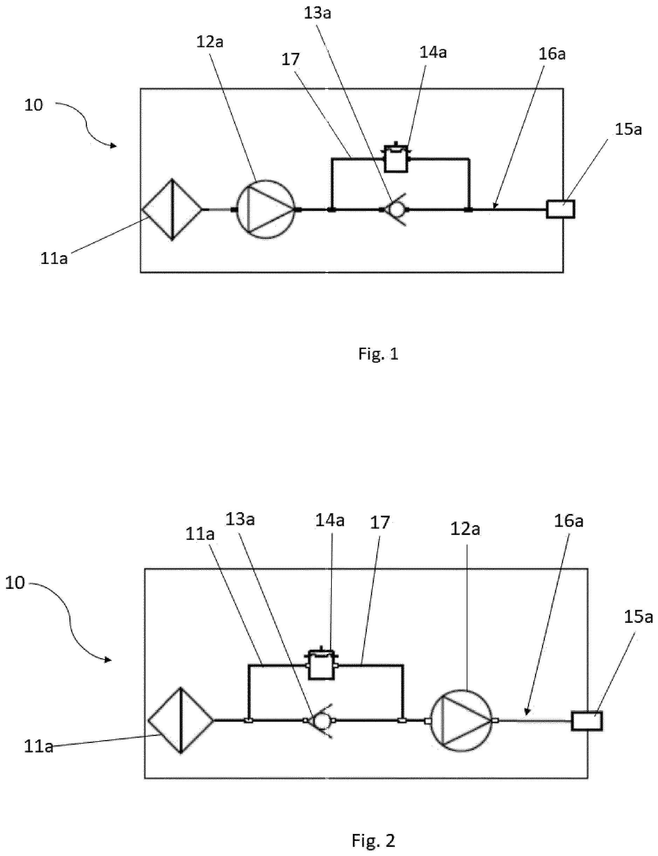

Figur 1 zeigt einen schematischen Aufbau einer Ausführungsform eines Beatmungsgerätes 10 mit einem inspiratorischen Atemgasweg 16a mit einem Bypass 17 und das Rückschlagventil 13a. Der inspiratorische Atemgasweg 16a umfasst einen Geräteeingang 11a und einen Geräteausgang 15a. Der Geräteausgang 15a ist dabei patientennah angeordnet und der Geräteeingang 11a ist patientenfern angeordnet. Von dem Geräteeingang 11a erstreckt sich zu dem Geräteausgang 15a der Atemgasweg 16a. Der Atemgasweg 16a umfasst einen Atemgasantrieb 12a, ein Rückschlagventil 13a und ein Schaltventil 14a. Das Schaltventil 14a ist in einem Bypass 17 zu dem Rückschlagventil 13a angeordnet. Der Bypass 17 zweigt zwischen dem Atemgasantrieb 12a und dem Rückschlagventil 13a ab und mündet zwischen dem Rückschlagventil 13a und dem Geräteausgang 15a wieder in den inspiratorischen Atemgasweg 16a. Der Atemgasantrieb 12a ist zwischen dem Geräteeingang 11a und dem Rückschlagventil 13a angeordnet. Über das im Bypass 17 angeordnete Schaltventil 14a kann Atemgas in Richtung des Geräteeingangs 11a rückgeführt werden, wodurch das Rückschlagventil 13a umgangen wird.

Figur 2 zeigt eine alternative Anordnung der in Figur 1 gezeigten Ausführungsform des Beatmungsgerätes 10 mit dem inspiratorischen Atemgasweg 16a mit dem Bypass 17 um dem Rückschlagventil 13a. Der inspiratorische Atemgasweg 16a umfasst den Geräteeingang 11a und den Geräteausgang 15a. Von dem Geräteeingang 11a erstreckt sich zu dem Geräteausgang 15a der Atemgasweg 16a. Der Atemgasweg 16a umfasst das Rückschlagventil 13a, den Atemgasantrieb 12a und das Schaltventil 14a. Das Schaltventil 14a ist in dem Bypass 17 zu dem Rückschlagventil 13a angeordnet. Der Bypass 17 zweigt in dieser alternativen Anordnung zwischen dem Geräteeingang 11a und dem Rückschlagventil 13a ab und mündet zwischen dem Rückschlagventil 13a und dem Atemgasantrieb 12a wieder in den inspiratorischen Atemgasweg 16a. Der Atemgasantrieb 12a ist zwischen dem Rückschlagventil 13a und dem Geräteausgang 15a angeordnet. Über das im Bypass 17 angeordnete Schaltventil 14a kann Atemgas in Richtung des Geräteeingangs 11a rückgeführt werden, nachdem es den Atemgasantrieb 12a passiert hat.

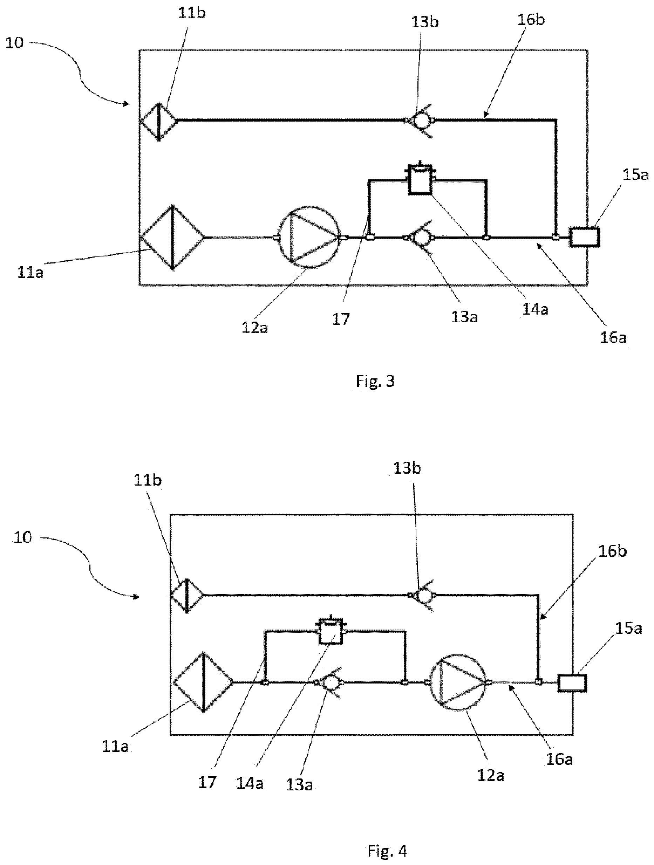

Figur 3 zeigt einen schematischen Aufbau einer weiteren Ausführungsform des in Figur 1 gezeigten Beatmungsgerätes 10, mit dem inspiratorischen Atemgasweg 16a, dem Bypass 17 und einem zweiten inspiratorischen Atemgasweg 16b. Der inspiratorische Atemgasweg 16a erstreckt sich von dem Geräteeingang 11a zu dem Geräteausgang 15a. Der inspiratorische Atemgasweg 16a umfasst den Atemgasantrieb 12a, das Rückschlagventil 14a und das Schaltventil 14a, wobei das Schaltventil 14a in dem Bypass 17 zur Umgehung des Rückschlagventils 13a angeordnet ist. Die in Figur 3 gezeigte Ausführungsform des Beatmungsgerätes 10 weist einen zweiten inspiratorischen Atemgasweg 16b auf, der sich von einem zweiten Geräteeingang 11b zu dem gemeinsamen Geräteausgang 15a erstreckt. Der zweite inspiratorische Atemgasweg 16b mündet zwischen dem Rückschlagventil 13a und dem gemeinsamen Geräteausgang 15a in den inspiratorischen Atemgasweg 16a ein. Der zweite inspiratorische Atemgasweg 16b kann ein Rückschlagventil 13b umfassen. Über den zweiten inspiratorischen Atemgasweg 16b kann beispielsweise bei einer Blockade/Störung des inspiratorischen Atemgasweges 16a Atemgas über den separaten Geräteeingang 11b bezogen werden und dem Patienten zugeführt werden. Zusätzlich kann bei einer Blockade eines exspiratorischen Atemgasweges Atemgas durch Umgehung des Rückschlagventils 13a durch das Schaltventil 14a rückgeführt werden und dem Patienten eine Ausatmung ermöglicht werden.

Figur 4 zeigt eine alternative Anordnung der in Figur 2 gezeigten Ausführungsform des Beatmungsgerätes 10, mit dem inspiratorischen Atemgasweg 16a, dem Bypass 17 und dem zweiten inspiratorischen Atemgasweg 16b. Bei dieser alternativen Ausführungsform ist in dem inspiratorischen Atemgasweg 16a das Rückschlagventil 13a zwischen dem Geräteeingang 11a und dem Atemgasantrieb 12a angeordnet. Um das Rückschlagventil 13a ist der Bypass 17 ausgebildet, der zwischen dem Geräteeingang 11a abzweigt und zwischen dem Rückschlagventil 13a und dem Atemgasantrieb 12a wieder in den inspiratorischen Atemgasweg 16a einmündet. Der Atemgasantrieb 12a ist zwischen dem Rückschlagventil 13a und dem Geräteausgang 15a angeordnet. Der zweite inspiratorische Atemgasweg 16b mündet zwischen dem Atemgasantrieb 12a und dem gemeinsamen Geräteausgang 15a in den inspiratorischen Atemgasweg 16a ein. Der zweite inspiratorische Atemgasweg 16b kann ein Rückschlagventil 13b umfassen. Über den zweiten inspiratorischen Atemgasweg 16b kann auch bei dieser Ausführungsform beispielsweise bei einer Blockade/Störung des inspiratorischen Atemgasweges 16a Atemgas über den separaten Geräteeingang 11b bezogen werden und dem Patienten zugeführt werden. Zusätzlich kann ebenfalls bei einer Blockade eines exspiratorischen Atemgasweges Atemgas durch Umgehung des Rückschlagventils 13a durch das Schaltventil 14a rückgeführt werden und dem Patienten eine Ausatmung ermöglicht werden.

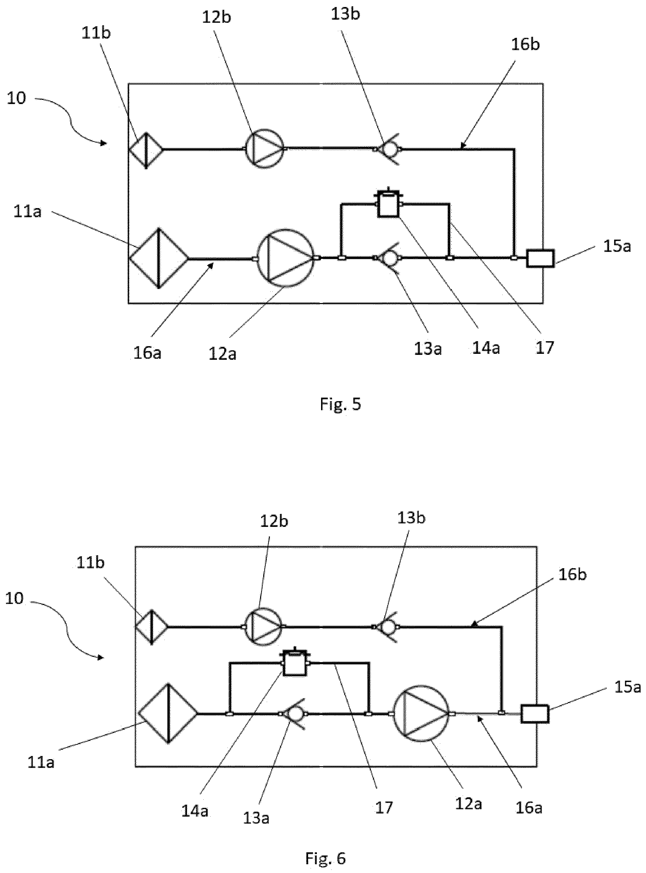

Figur 5 zeigt eine alternative Anordnung der in Fig. 1 und Fig. 3 gezeigten Ausführungsform des Beatmungsgerätes 10, mit dem inspiratorischen Atemgasweg 16a, dem Bypass 17 und dem zweiten inspiratorischen Atemgasweg 16b.

Der inspiratorische Atemgasweg 16a erstreckt sich von dem Geräteeingang 11a zu dem Geräteausgang 15a und umfasst den Atemgasantrieb 12a, das Rückschlagventil 14a und das Schaltventil 14a. Das Schaltventil 14a ist in dem Bypass 17 zur Umgehung des Rückschlagventils 13a angeordnet. Der Bypass 17 ist zwischen dem Atemgasantrieb 12a und dem Geräteausgang 15a ausgebildet. Die in Figur 5 gezeigte Ausführungsform des Beatmungsgerätes 10 weist ebenfalls den zweiten inspiratorischen Atemgasweg 16b auf, der sich von dem zweiten Geräteeingang 11b zu dem gemeinsamen Geräteausgang 15a erstreckt. Der zweite inspiratorische Atemgasweg 16b mündet zwischen dem Rückschlagventil 13b und dem gemeinsamen Geräteausgang 15a in den inspiratorischen Atemgasweg 16a ein. Der zweite inspiratorische Atemgasweg 16b weist ein Rückschlagventil 13b und einen Atemgasantrieb 12b auf, wobei der Atemgasantrieb 12b zwischen dem zweiten Geräteeingang 11b und dem Rückschlagventil 13b angeordnet ist. Über den zweiten inspiratorischen Atemgasweg 16b kann auch bei dieser Ausführungsform beispielsweise bei einer Blockade/Störung des inspiratorischen Atemgasweges 16a Atemgas über den separaten Geräteeingang 11b bezogen werden und dem Patienten zugeführt werden. Zusätzlich kann ebenfalls bei einer Blockade eines exspiratorischen Atemgasweges Atemgas durch Umgehung des Rückschlagventils 13a über das im Bypass 17 angeordnete Schaltventil 14a rückgeführt werden und dem Patienten eine Ausatmung ermöglicht werden.

Figur 6 zeigt eine alternative Anordnung der in Figur 2 und Figur 4 gezeigten Ausführungsform des Beatmungsgerätes 10, mit dem inspiratorischen Atemgasweg 16a, dem Bypass 17 und dem zweiten inspiratorischen Atemgasweg 16b. Der inspiratorische Atemgasweg 16a erstreckt sich von dem Geräteeingang 11a zu dem Geräteausgang 15a und umfasst das Rückschlagventil 14a, das Schaltventil 14a und den Atemgasantrieb 12a. Das Schaltventil 14a ist in dem Bypass 17 zur Umgehung des Rückschlagventils 13b angeordnet. Der Bypass 17 mit dem Schaltventil 14a ist zwischen dem Geräteingang 11a und dem Atemgasantrieb 12a angeordnet. Die in Figur 6 gezeigte Ausführungsform des Beatmungsgerätes 10 weist den zweiten inspiratorischen Atemgasweg 16b auf, der sich von einem zweiten Geräteeingang 11b zu dem gemeinsamen Geräteausgang 15a erstreckt. Der zweite inspiratorische Atemgasweg 16b mündet zwischen dem Atemgasantrieb 12a und dem gemeinsamen Geräteausgang 15a in den inspiratorischen Atemgasweg 16a ein. Der zweite inspiratorische Atemgasweg 16b weist ein Rückschlagventil 13b und einen Atemgasantrieb 12b auf, wobei der Atemgasantrieb 12b zwischen dem zweiten Geräteeingang 11b und dem Rückschlagventil 13b angeordnet ist.

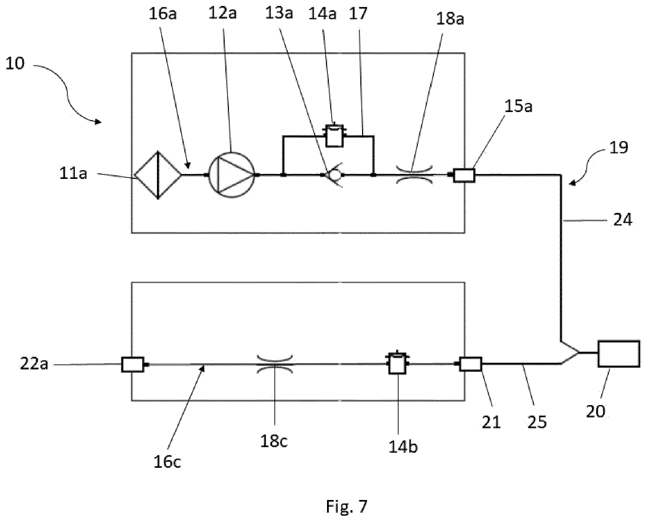

Figur 7 einen schematischen Aufbau einer erfindungsgemäßen Ausführungsform des Beatmungsgerätes 10 mit dem inspiratorischen Atemgasweg 16a mit einem Bypass 17 und einem exspiratorischen Atemgasweg 16c.

Der inspiratorische Atemgasweg 16a erstreck sich von dem Geräteeingang 11a hin zu dem Geräteausgang 15a. Der inspiratorische Atemgasweg 16a umfasst dabei den Atemgasantrieb 12a sowie das Rückschlagventil 13a und das Schaltventil 14a, welches in einem Bypass 17 zu dem Rückschlagventil 13a angeordnet ist sowie eine Flussmesseinrichtung 18a. Die Flussmesseinrichtung 18a ist zwischen dem Bypass 17 und dem Geräteausgang 15a angeordnet. Über die Flussmesseinrichtung 18a wird ein Volumen des Atemgases im Atemgasweg erfasst. Bei einem vorgegebenen Volumen an Atemgas über einen vorgegebenen Zeitraum ist das Schaltventil beispielsweise mittels eines 3/2-Wegeventils schaltbar. Das Schaltventil ist in der Regel eingerichtet und ausgebildet einen PEEP (positive end-expiratory pressure) im Bereich von 0-20hPa, bevorzugt 0-15hPa einzustellen.

Der exspiratorische Atemgasweg 16c erstreckt sich von einem exspiratorischen Geräteeingang 21 zu dem exspiratorischen Geräteausgang 22a und umfasst ein Schaltventil 14b und eine Flussmesseinrichtung 18c. Die Flussmesseinrichtung 18c ist dabei zwischen dem exspiratorischen Geräteausgang 22a und dem Schaltventil 14b angeordnet. Die Flussmesseinrichtung 18c dient dabei der Erfassung eines Flusses bzw. eines Volumens oder eines Druckes des Atemgases im Atemgasweg. Basierend auf den durch die Flussmesseinrichtung 18c erfassten Werte kann eine Rückmeldung über das Volumen des durch den Patienten abgegebenen Atemgases an das Beatmungsgerät erfolgen.

An den Geräteausgang 15a ist ein Schlauchsystem 19 mit einem ersten Zweig 24 und einem zweiten Zweig 25 adaptiert. Der erste Zweig 24 führt von dem Geräteausgang 15a zu einem Patienteninterface 20. Vor dem Patienteninterface 20 führt das Schlauchsystem 19 mit einem zweiten Zweig 25 zu dem exspiratorischen Geräteeingang 21 für exspiratorisches Atemgas. Über das Schlauchsystem 19 ist ein Patienteninterface 20 mit dem inspiratorischen Zweig 16a und dem exspiratorischen Zweig 16c verbunden.

Bei der in Figur 7 gezeigten Ausführungsform des Beatmungsgerätes 10 kann ein Atemgas über den Geräteeingang 11a bezogen werden und über den Atemgasantrieb 12a über das Rückschlagventil 13a in Richtung des Geräteausgangs 15a geführt werden. Das Atemgas wird von dem Geräteausgang 15a über das den Zweig 24 des adaptierten Schlauchsystems 19 zu dem Patienteninterface 20 weitergeleitet, worüber ein Patient das Atemgas einatmen kann. Die ausgeatmete Luft des Patienten kann über den Zweig 25 des adaptierten Schlauchsystems 19 über den exspiratorischen Geräteeingang 21 dem Atemgasweg 16c zugeführt werden. Über den exspiratorischen Atemgasweg 16c wird das Atemgas hin zu dem exspiratorischen Geräteausgang 22a geführt und an die Umgebung abgegeben. Bei einer Blockade/Störung des exspiratorischen Atemgasweges 16c kann das Atemgas durch Öffnung des Schaltventils 14a in dem Bypass 17 in dem inspiratorischen Atemgasweg 16a abgeführt werden.

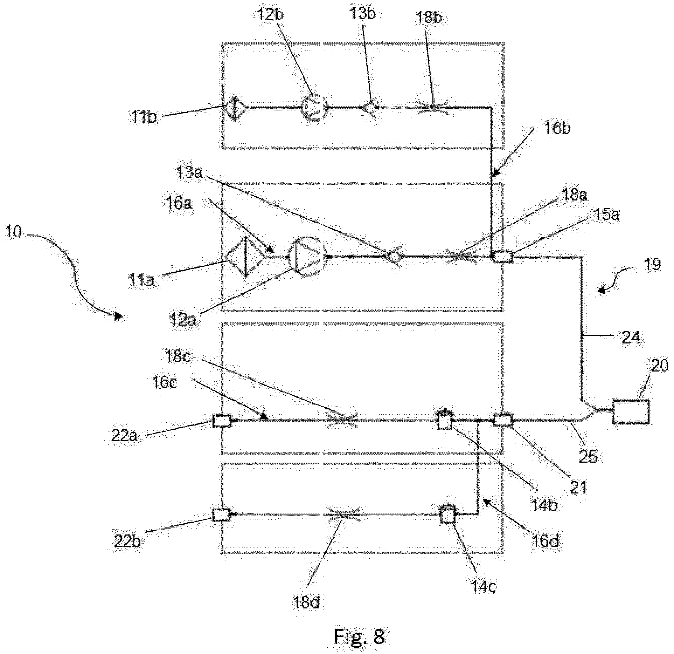

Figur 8 zeigt einen schematischen Aufbau einer weiteren Ausführungsform des Beatmungsgerätes 10 mit dem ersten inspiratorischen Atemgasweg 16a und dem zweiten inspiratorischen Atemgasweg 16b sowie mit dem exspiratorischen Atemgasweg 16c und einem separaten exspiratorischen Atemgasweg 16d.

Der inspiratorische Atemgasweg 16a erstreckt sich von dem Geräteeingang 11a hin zu dem Geräteausgang 15a. Der inspiratorische Atemgasweg 16a umfasst den Atemgasantrieb 12a, das Rückschlagventil 13a sowie eine Flussmesseinrichtung 18a. Die Flussmesseinrichtung 18a ist zwischen dem Rückschlagventil 13a und dem Geräteausgang 15a angeordnet. Die Flussmesseinrichtung 18a erfasst einen Fluss bzw. ein Volumen oder einen Druck des Atemgases welches über den inspiratorischen Atemgasweg 16a an den Patienten abgegeben wird.

Die in Figur 8 gezeigte Ausführungsform des Beatmungsgerätes 10 weist einen zweiten inspiratorischen Atemgasweg 16b auf, der sich von dem zweiten Geräteeingang 11b zu dem gemeinsamen Geräteausgang 15a erstreckt. Der zweite inspiratorische Atemgasweg 16b mündet zwischen der Flussmesseinrichtung und dem gemeinsamen Geräteausgang 15a in den inspiratorischen Atemgasweg 16a ein. Der zweite inspiratorische Atemgasweg 16b kann den separaten Atemgasantrieb 12b, das Rückschlagventil 13b und eine Flussmesseinrichtung 18b umfassen. Der separate Atemgasantrieb 12b ist zwischen dem zweiten Geräteeingang 11b und dem Rückschlagventil 13b angeordnet. Die Flussmesseinrichtung 18b kann ein Volumen oder Druck des inspiratorischen Atemgases in dem Atemgasweg 16b an erfassen und an das Beatmungsgerät 10 übermitteln. Über den zweiten inspiratorischen Atemgasweg 16b kann bei einer Blockade des inspiratorischen Atemgasweges 16a Atemgas über den Geräteeingang 11b bezogen und dem Patienten zugeführt werden.

Die in Figur 8 gezeigte Ausführungsform des Beatmungsgerätes 10 weist ferner den exspiratorische Atemgasweg 16c auf, der sich von dem exspiratorischen Geräteeingang 21 zu dem exspiratorischen Geräteausgang 22a erstreckt und das Schaltventil 14b und die Flussmesseinrichtung 18c umfasst. Die Flussmesseinrichtung 18c ist dabei zwischen dem exspiratorischen Geräteausgang 22a und dem Schaltventil 14b angeordnet. Die Flussmesseinrichtung 18c erfasst ein Volumen und/oder einen Druck des Atemgases, welches der Patient abgibt. Über den exspiratorischen Atemgasweg 16c ist ein von dem Patienten abgegebenes Atemgas abführbar.

Die Flussmesseinrichtungen 18a, 18b, 18c und 18d können eingerichtet sein, einen Fluss bzw. ein Volumen oder einen Druck des Atemgases in dem Atemgasweg zu erfassen und basierend auf diesem ein Schaltventil zu schalten bzw. zu öffnen und zu schließen oder eine Rückmeldung an das Beatmungsgerät 10 über den Fluss bzw. das Volumen oder den Druck des dem Patienten zugeführten Atemgases oder von dem Patienten abgegebenes Atemgases zu liefern.

Die in Figur 8 gezeigte Ausführungsform des Beatmungsgerätes 10 weist zudem einen separaten exspiratorischen Atemgasweg 16d auf. Der separate exspiratorische Atemgasweg 16d zweigt von dem exspiratorischen Atemgasweg 16c ab und erstreckt sich hin zu einem separaten Geräteausgang 22b. Der separate exspiratorische Atemgasweg 16d umfasst ein Schaltventil 14c und eine Flussmesseinrichtung 18d. Die Flussmesseinrichtung 18d erfasst einen Fluss bzw. ein Volumen oder einen Druck des Atemgases, welches der Patient abgibt. Über den separaten exspiratorischen Atemgasweg 16d ist bei einer Blockade/Störung des exspiratorischen Atemgaswegs 16c von dem Patienten abgegebenes Atemgas abführbar.

An den Geräteausgang 15a ist ein Schlauchsystem 19 mit einem ersten Zweig 24 und einem zweiten Zweig 25 adaptiert. Der erste Zweig 24 führt von dem Geräteausgang 15a zu einem Patienteninterface 20. Vor dem Patienteninterface 20 führt das Schlauchsystem 19 mit dem zweiten Zweig 25 zu dem exspiratorischen Geräteeingang 21 für exspiratorisches Atemgas. Über das Schlauchsystem 19 ist das Patienteninterface 20 mit dem inspiratorischen Atemgasweg 16a, dem zweiten inspiratorischen Atemgasweg 16b, dem exspiratorischen Atemgasweg 16c und dem separaten exspiratorischen Atemgasweg 16d verbunden.

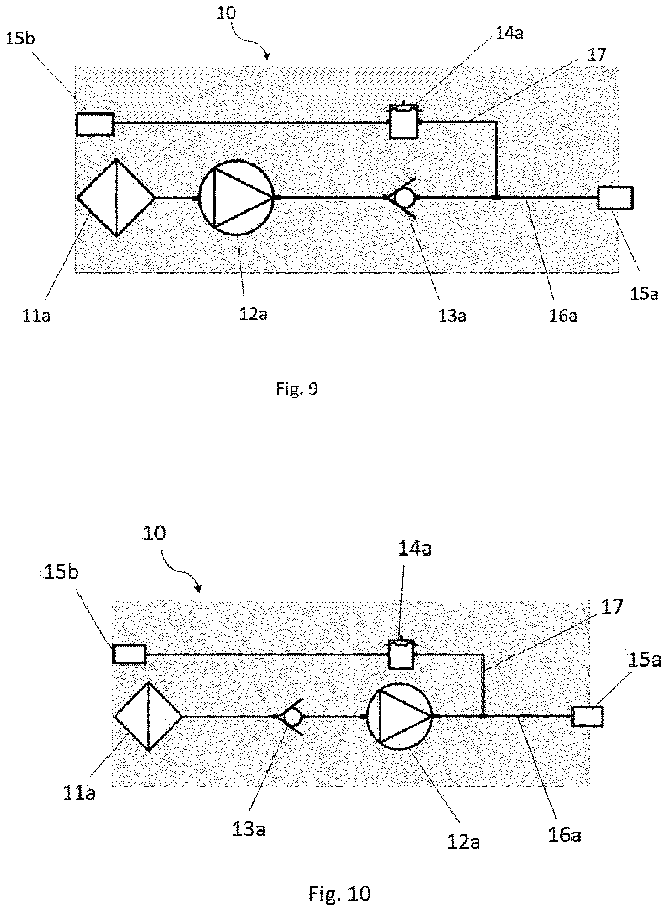

Figur 9 zeigt einen schematischen Aufbau einer alternativen Ausführungsform des Beatmungsgerätes 10 mit dem inspiratorischen Atemgasweg 16a mit einem offenen Bypass 17 mit einem separaten Geräteausgang 15b. Der inspiratorische Atemgasweg 16a des Beatmungsgerätes 10 umfasst den Geräteeingang 11a und den Geräteausgang 15a. Zwischen dem Geräteeingang 11a und dem Geräteausgang 15a sind der Atemgasantrieb 12a, das Rückschlagventil 13a sowie das Schaltventil 14a angeordnet, wobei das Schaltventil 14a in dem Bypass 17 angeordnet ist. Der Bypass 17 ist als offener Bypass ausgebildet, der einen separaten Geräteausgang 15b umfasst. Der offene Bypass 17 zweigt von dem inspiratorischen Atemgasweg 16a zwischen dem Rückschlagventil 13a und dem Geräteausgang 15a ab und mündet in dem separaten Geräteausgang 15b. Der Atemgasantrieb 12a ist zwischen dem Geräteeingang 11 und dem Rückschlagventil 13a angeordnet. Das Schaltventil 14a kann zeitgesteuert, patientengetriggert oder basierend auf einem durch eine Flussmesseinrichtung erfassten Fluss bzw. Volumen des Atemgases schaltbar sein. Die Flussmessrichtung ist dabei zwischen dem separaten Geräteausgang 15b und dem Schaltventil 14a angeordnet sein. Über das in dem offenen Bypass 17 angeordnete Schaltventil 14a ist es möglich, bei einer Blockade des inspiratorischen Atemgaswegs 16a von dem Patienten ausgegebenes Atemgas über das Schaltventil 14a abzugeben. Durch die Anordnung des Rückschlagventils 13a zwischen dem Atemgasantrieb 12a und dem Geräteausgang 15a, ist das Rückschlagventil 13a auf der Druckseite des Beatmungsgerätes 10 angeordnet.

Figur 10 zeigt einen alternativen schematischen Aufbau einer in Fig. 9 gezeigten Ausführungsform des Beatmungsgerätes 10 mit dem inspiratorischen Atemgasweg 16a und dem offenen Bypass 17 mit dem separaten Geräteausgang 15b. Der inspiratorische Atemgasweg 16a des Beatmungsgerätes 10 umfasst dabei den Geräteeingang 11a und den Geräteausgang 15a. Zwischen dem Geräteeingang 11a und dem Geräteausgang 15a ist das Rückschlagventil 13a, der Atemgasantrieb 12a sowie das Schaltventil 14a angeordnet, wobei das Schaltventil 14a in dem Bypass 17 angeordnet ist. Der Bypass 17 ist als offener Bypass ausgebildet, der einen separaten Geräteausgang 15b umfasst. Der offene Bypass 17 zweigt von dem inspiratorischen Atemgasweg 16a zwischen dem Atemgasantrieb 12a und dem Geräteausgang 15a ab und mündet in dem separaten Geräteausgang 15b. Der Atemgasantrieb 12a ist zwischen dem Rückschlagventil 13a und dem Geräteausgang 15a angeordnet. Über das in dem offenen Bypass 17 angeordnete Schaltventil 14a ist es möglich, bei einer Blockade des inspiratorischen Atemgaswegs 16a von dem Patienten ausgegebenes Atemgas über das Schaltventil 14a abzugeben. Durch die Anordnung des offenen Bypasses 17 zwischen dem Atemgasantrieb 12a und dem Geräteausgang 15a kann Atemgas rückgeführt werden, ohne den Atemgasantrieb passieren zu müssen. Durch die Anordnung des Rückschlagventils 13a zwischen dem Geräteeingang 11a und dem Atemgasantrieb 12a, ist das Rückschlagventil 13a auf der Saugseite des Beatmungsgerätes 10 angeordnet.

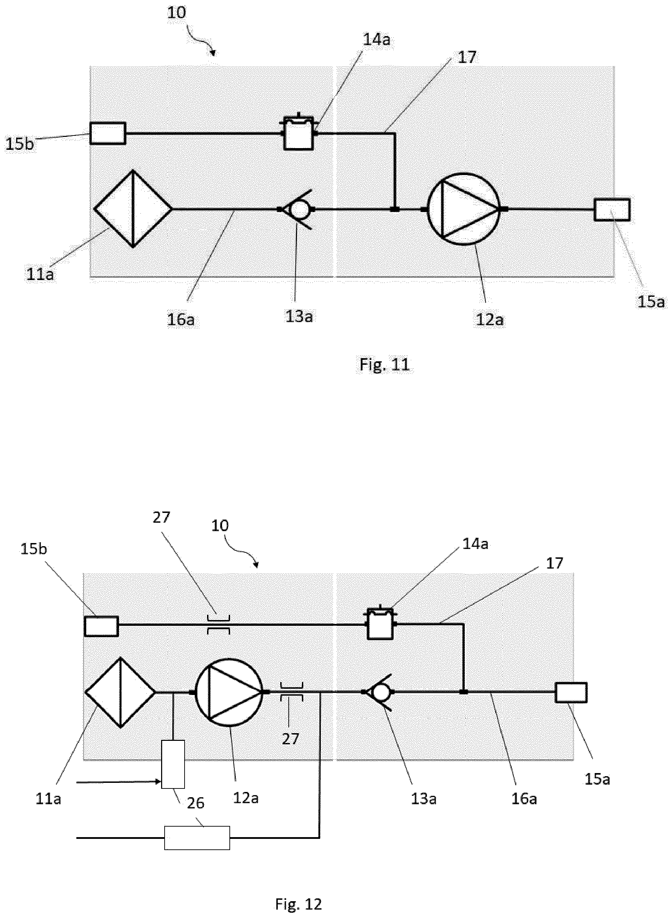

Figur 11 zeigt einen alternativen schematischen Aufbau einer in den Figuren 9 und 10 gezeigten alternativen Ausführungsform des Beatmungsgerätes 10 mit dem inspiratorischen Atemgasweg 16a und dem offenen Bypass 17 mit dem separaten Geräteausgang 15b. Der inspiratorische Atemgasweg 16a des Beatmungsgerätes 10 umfasst dabei den Geräteeingang 11a und den Geräteausgang 15a. Zwischen dem Geräteeingang 11a und dem Geräteausgang 15a ist der Atemgasantrieb 12a, das Rückschlagventil 13 sowie das Schaltventil 14a, wobei das Schaltventil 14a in dem Bypass 17 angeordnet ist. Der Bypass 17 ist als offener Bypass ausgebildet, der einen separaten Geräteausgang 15b umfasst. Der offene Bypass 17 zweigt von dem inspiratorischen Atemgasweg 16a zwischen dem Rückschlagventil 13a und dem Atemgasantrieb 12a ab und mündet in dem separaten Geräteausgang 15b. Der Atemgasantrieb 12a ist zwischen dem Rückschlagventil 13a und dem Geräteausgang 15a angeordnet. Über das in dem offenen Bypass 17 angeordnete Schaltventil 14a ist es möglich, bei einer Blockade des inspiratorischen Atemgaswegs 16a von dem Patienten ausgegebenes Atemgas über das Schaltventil 14a abzugeben. Durch die Anordnung des Rückschlagventils 13a zwischen dem Geräteeingang 11a und dem Atemgasantrieb 12a, ist das Rückschlagventil 13a auf der Saugseite des Beatmungsgerätes 10 angeordnet.

Figur 12 zeigt einen schematischen Aufbau einer alternativen Ausführungsform des Beatmungsgerätes 10 mit dem inspiratorischen Atemgasweg 16a mit dem offenen Bypass 17 mit dem separaten Geräteausgang 15b. Der inspiratorische Atemgasweg 16a des Beatmungsgerätes 10 umfasst den Geräteeingang 11a und den Geräteausgang 15a. Zwischen dem Geräteeingang 11a und dem Geräteausgang 15a sind der Atemgasantrieb 12a, das Rückschlagventil 13a, eine Flussmesseinrichtung 27 sowie das Schaltventil 14a angeordnet, wobei das Schaltventil 14a in dem Bypass 17 angeordnet ist. Der Bypass 17 ist als offener Bypass ausgebildet, der einen separaten Geräteausgang 15b sowie zumindest eine Flussmesseinrichtung 27 umfasst. Der offene Bypass 17 zweigt von dem inspiratorischen Atemgasweg 16a zwischen dem Rückschlagventil 13a und dem Geräteausgang 15a ab und mündet in dem separaten Geräteausgang 15b. Der Atemgasantrieb 12a ist zwischen dem Geräteeingang 11a und dem Rückschlagventil 13a angeordnet. Das Schaltventil 14a kann zeitgesteuert, patientengetriggert oder basierend auf einen durch die in dem Bypass 17 angeordnete Flussmesseinrichtung 27 erfassten Fluss bzw. Volumen des Atemgases schaltbar sein. Die Flussmessrichtung 27 im Bypass 17 ist dabei zwischen dem separaten Geräteausgang 15b und dem Schaltventil 14a angeordnet. Über das in dem offenen Bypass 17 angeordnete Schaltventil 14a ist es möglich, bei einer Blockade des inspiratorischen Atemgaswegs 16a von dem Patienten ausgegebenes Atemgas über das Schaltventil 14a abzugeben. Durch die Anordnung des Rückschlagventils 13a zwischen dem Atemgasantrieb 12a und dem Geräteausgang 15a, ist das Rückschlagventil 13a auf der Druckseite des Beatmungsgerätes 10 angeordnet.

Die in Figur 12 gezeigte Ausführungsform zeigt ferner, dass der inspiratorische Atemgasweg 16a zusätzlich zumindest ein weiteres Ventil 26 umfasst, welches eingerichtet ist, eine Sauerstoff- oder Luft bzw. Druckluftzufuhr zu dem inspiratorischen Atemgasweg 16a zu gewährleisten. Zudem umfasst der inspiratorische Atemgasweg 16a zumindest eine Flussmesseinrichtung 27, wobei die Flussmesseinrichtung 27 zwischen dem Atemgasantrieb 12a und dem Rückschlagventil 13a angeordnet ist. Das weitere Ventil 26 kann zwischen dem Geräteeingang 11a und dem Atemgasantrieb 12a sowie zwischen der Flussmesseinrichtung 27 und dem Rückschlagventil 13a in den inspiratorischen Atemgasweg 16a einmünden. Das weitere Ventil 26 ist in der Regel ein Proportionalventil.

Bei der in der Figur 12 gezeigten Ausführungsform ist der Bereich des offenen Bypasses 17 mit dem Schaltventil 14a, der Flussmesseinrichtung 27 und dem separaten Geräteausgang 15b sowie der Bereich des inspiratorischen Atemgasweges 16a mit dem Rückschlagventil 13a und dem Geräteausgang 15a aus dem Beatmungsgerät entnehmbar und autoklavierbar. Der Bereich des inspiratorischen Atemgasweges 16a mit dem Geräteeingang 11a, dem Atemgasantrieb 12a und der Flussmesseinrichtung 27 ist derart eingerichtet und ausgebildet, dass dieser nicht mit kontaminiertem Atemgas in Kontakt kommt.

Optional können die in den Figuren 9 bis 11 gezeigten alternativen Ausführungsformen des Beatmungsgerätes 10 jeweils eine Flussmesseinrichtung umfassen, die einen Fluss bzw. ein Volumen oder einen Druck des Atemgases im Atemgasweg erfasst.

Optional sind kann das Schaltventil 14a in den alternativen Ausführungsformen, wie in den Figuren 9 bis 11 gezeigt, basierend auf dem durch die Flussmesseinrichtung erfassten Fluss bzw. Volumen schaltbar sein.

Optional können die in den Figuren 9 bis 11 gezeigten alternativen Ausführungsformen mit den in den Figuren 1 bis 8 gezeigten Ausführungsformen kombiniert werden. Alternativ kann der in den Figuren 9 bis 11 gezeigte offene Bypass 17 den geschlossenen Bypass 17 in den in den Figuren 1 bis 8 gezeigten Ausführungsformen ersetzen.

### Bezugszeichenliste

- 10: Beatmungsgerät
- 11a: Geräteeingang
- 11b: zweiter, separater Geräteeingang
- 12a: Atemgasantrieb
- 12b: Atemgasantrieb
- 13a: Rückschlagventil
- 13b: Rückschlagventil
- 14a: Schaltventil
- 14b: Schaltventil
- 14c: Schaltventil
- 14d: Schaltventil
- 15a: Geräteausgang
- 15b: separater Geräteausgang
- 16a: erster Atemgasweg
- 16b: zweiter Atemgasweg
- 16c: Atemgasweg, für den exspiratorischen Atemgasfluss
- 16d: separater exspiratorischer Atemgasweg
- 17: Bypass
- 18a: Flussmesseinrichtung
- 18b: Flussmesseinrichtung
- 18c: Flussmesseinrichtung
- 18d: Flussmesseinrichtung
- 19: Schlauchsystem
- 20: Patienteninterface
- 21: exspiratorischer Geräteeingang
- 22a: exspiratorischer Geräteausgang
- 22b: exspiratorischer Geräteausgang
- 23: Schlauchsystem
- 24: erster Zweig
- 25: zweiter Zweig
- 26: weitere Ventile, Proportionalventil
- 27: weitere Flussmesseinrichtungen

## Patentansprüche

1. Beatmungsgerät (10) das mindestens einen exspiratorischen Atemgasweg umfasst, mit mindestens einem Geräteeingang (11a) und einem Geräteausgang (15a) und einem inspiratorischen Atemgasweg (16a) zwischen Geräteeingang (11a) und Geräteausgang (15a), wobei im inspiratorischen Atemgasweg (16a) ein Atemgasantrieb (12a), ein Rückschlagventil (13a) und ein Schaltventil (14a) angeordnet sind, wobei das Rückschlagventil (13a) in dem inspiratorischen Atemgasweg (16a) angeordnet ist und eingerichtet ist einen Atemgasstrom in einer Richtung vom Geräteausgang (15a) zum Geräteeingang (11a) zu verhindern und wobei das Schaltventil (14a) eingerichtet ist bei einer Blockade des exspiratorischen Atemgasweges, Atemgas des Patienten über den inspiratorischen Atemgasweg (16a) abzuführen, indem durch das Schaltventil (14a) das Rückschlagventil (13a) umgangen wird und dafür einen Atemgasstrom in einer Richtung vom Geräteausgang (15a) zum Geräteeingang (11a) oder zu einer separaten Geräteöffnung zumindest zeitweise zu ermöglichen, wobei das Schaltventil (14a) in einem Bypass (17) zu dem Rückschlagventil (13a) angeordnet ist,
**dadurch gekennzeichnet, dass**
zwischen dem Rückschlagventil (13a) und dem Geräteausgang (15a) eine Flussmesseinrichtung (18a) angeordnet ist, dass die Flussmesseinrichtung (18a) eingerichtet ist ein Volumen des Atemgases im Atemgasweg zu erfassen, und dass das Schaltventil (14a) eingerichtet ist, basierend auf dem erfassten Volumen des Atemgases öffenbar oder schließbar zu sein, was eine atemgasvolumenabhängige Schaltung des Schaltventils (14a) ermöglicht.

2. Beatmungsgerät (10) nach Anspruch 1, **dadurch gekennzeichnet, dass** das mindestens eine Schaltventil (14a) einen Bypass (17) um das Rückschlagventil (13a) ausbildet.

3. Beatmungsgerät (10) nach zumindest einem der voranstehenden Ansprüche, **dadurch gekennzeichnet, dass** das mindestens eine Schaltventil (14a) eingerichtet ist, den Bypass (17) zu öffnen und / oder zu schließen, wobei das Schaltventil (14a) automatisch schaltbar eingerichtet ist

4. Beatmungsgerät (10) nach zumindest einem der voranstehenden Ansprüche, **dadurch gekennzeichnet, dass** der Atemgasantrieb (12a) zwischen dem Geräteeingang (11a) und dem Rückschlagventil (13a) angeordnet ist und eingerichtet ist, Atemgas in Richtung des Rückschlagventils (13a) und des Geräteausgangs (15a) zu fördern.

5. Beatmungsgerät (10) nach zumindest einem der voranstehenden Ansprüche 1 bis 4, **dadurch gekennzeichnet, dass** der Bypass (17) zu dem Schaltventil (14a) zwischen dem Atemgasantrieb (12a) und dem Rückschlagventil (13a) abzweigt und zwischen dem Rückschlagventil (13a) und dem Geräteausgang (15a) wieder in den Atemgasweg (16a) mündet.

6. Beatmungsgerät (10) nach einem der Ansprüche 1 bis 3, **dadurch gekennzeichnet, dass** der Atemgasantrieb (12a) zwischen dem Rückschlagventil (13a) und dem Geräteausgang (15a) angeordnet ist und eingerichtet ist, Atemgas in Richtung des Geräteausgangs (15a) zu fördern.

7. Beatmungsgerät (10) nach Anspruch 6, **dadurch gekennzeichnet, dass** der Bypass (17) zu dem Schaltventil (14a) zwischen dem Geräteeingang (11a) und dem Rückschlagventil (13a) abzweigt und zwischen dem Rückschlagventil (13a) und dem Atemgasantrieb (12a) wieder in den inspiratorischen Atemgasweg (16a) mündet.

8. Beatmungsgerät (10) nach zumindest einem der voranstehenden Ansprüche, **dadurch gekennzeichnet, dass** zusätzlich zu dem Bypass (17) ein zweiter inspiratorischer Atemgasweg (16b) ausgebildet ist, der einen zweiten Geräteeingang (11b) und mindestens ein Rückschlagventil (13b) umfasst und vor dem gemeinsamen Geräteausgang (15a) in den ersten inspiratorischen Atemgasweg (16a) mündet.

9. Beatmungsgerät (10) nach zumindest einem der voranstehenden Ansprüche, **dadurch gekennzeichnet, dass** ein Atemgasweg (16c) für einen exspiratorischen Atemgasfluss umfasst ist, der einen exspiratorischen Geräteeingang (21) und einen exspiratorischen Geräteausgang (22a) umfasst.

10. Beatmungsgerät (10) nach Anspruch 9, **dadurch gekennzeichnet, dass** von dem exspiratorischen Geräteeingang (21) der Atemgasweg (16c) für den exspiratorischen Atemgasfluss zu dem exspiratorischen Geräteausgang (22a) führt und in dem Atemgasweg (16c) für den exspiratorischen Atemgasfluss ein Schaltventil (14b) und eine Flussmesseinrichtung (18c) angeordnet sind.

11. Beatmungsgerät (10) nach Anspruch 10, **dadurch gekennzeichnet, dass** zwischen dem exspiratorischen Geräteeingang (21) und dem Schaltventil (14b) ein separater exspiratorischer Atemgasweg (16d) mit einem Schaltventil (14c) und einer Flussmesseinrichtung (18d) zu einem separaten Geräteausgang (22b) führt.

12. Beatmungsgerät (10) nach zumindest einem der voranstehenden Ansprüche, **dadurch gekennzeichnet, dass** an den Geräteausgang (15a) ein Schlauchsystem (19) adaptiert ist, das mit einem ersten Zweig (24) zu einem Patienteninterface (20) führt und vor dem Patienteninterface (20) mit einem zweiten Zweig (25) zu dem exspiratorischen Geräteeingang (21) für exspiratorisches Atemgas führt.

13. Beatmungsgerät (10) nach zumindest einem der Ansprüche 1-4 oder 6, **dadurch gekennzeichnet, dass** das Schaltventil (14a) in dem Bypass (17) angeordnet ist, wobei der Bypass (17) als offener Bypass ausgebildet ist, der einen separaten Geräteausgang (15b) umfasst, wobei der offene Bypass (17) von dem inspiratorischen Atemgasweg (16a) zwischen dem Rückschlagventil (13a) und dem Geräteausgang (15a) abzweigt und in den separaten Geräteausgang (15b) mündet, wobei der inspiratorische Atemgasweg (16a) zumindest ein weiteres Ventil (26) und eine Flussmesseinrichtung (27) aufweist, wobei die Flussmesseinrichtung (27) zwischen dem Atemgasantrieb (12a) und dem Rückschlagventil (13b) angeordnet ist.

14. Beatmungsgerät (10) nach zumindest einem der voranstehenden Ansprüche 1-12, **dadurch gekennzeichnet, dass** der Bypass geschlossen ist und in den Atemgasweg (16a) einmündet von dem er abzweigt und dabei das Rückschlagventil (13a) umgeht.

15. Beatmungsgerät (10) nach Anspruch 8, **dadurch gekennzeichnet, dass** sich der zweite inspiratorische Atemgasweg (16b), von dem zweiten Geräteeingang (11b) zu dem gemeinsamen Geräteausgang (15a) erstreckt, wobei der zweite inspiratorische Atemgasweg (16b) zwischen dem Rückschlagventil (13a) und dem gemeinsamen Geräteausgang (15a) in den inspiratorischen Atemgasweg (16a) einmündet, wobei über den zweiten inspiratorischen Atemgasweg (16b) bei einer Blockade/Störung des inspiratorischen Atemgasweges (16a) Atemgas über den separaten Geräteeingang (11b) bezogen werden und dem Patienten zugeführt werden kann und zusätzlich bei einer Blockade eines exspiratorischen Atemgasweges Atemgas, durch Umgehung des Rückschlagventils (13a), durch das Schaltventil (14a) rückgeführt werden und dem Patienten eine Ausatmung ermöglicht werden kann.

## Claims

1. A respiratory apparatus (10) comprising at least one expiratory respiratory gas path, having at least one apparatus inlet (11a) and one apparatus outlet (15a) and an inspiratory respiratory gas path (16a) between the apparatus inlet (11a) and the apparatus outlet (15a), wherein a respiratory gas drive (12a), a check valve (13a), and a switching valve (14a) are arranged in the inspiratory respiratory gas path (16a), wherein the check valve (13a) is arranged in the inspiratory respiratory gas path (16a) and is configured to prevent a flow of respiratory gas in a direction from the apparatus outlet (15a) to the apparatus inlet (11a), and wherein the switching valve (14a) is configured, when the expiratory respiratory gas path is blocked, to discharge respiratory gas of the patient via the inspiratory respiratory gas path (16a) in that the check valve (13a) is bypassed through the switching valve (14a) in order to at least temporarily enable a flow of respiratory gas in a direction from the apparatus outlet (15a) to the apparatus inlet (11a) or to a separate apparatus opening, wherein the switching valve (14a) is arranged in a bypass (17) to the check valve (13a), **characterized in that** a flow measuring device (18a) is arranged between the check valve (13a) and the apparatus outlet (15a), the flow measuring device (18a) is configured to record a volume of the respiratory gas in the respiratory gas path, and the switching valve (14a) is configured to be openable or closable based on the recorded volume of the respiratory gas, which enables the switching valve (14a) to be switched depending on the respiratory gas volume.

2. The respiratory apparatus (10) according to claim 1, **characterized in that** the at least one switching valve (14a) forms a bypass (17) around the check valve (13a).

3. The respiratory apparatus (10) according to at least one of the preceding claims, **characterized in that** the at least one switching valve (14a) is configured to open and/or to close the bypass (17), wherein the switching valve (14a) is configured to be switchable automatically.

4. The respiratory apparatus (10) according to at least one of the preceding claims, **characterized in that** the respiratory gas drive (12a) is arranged between the apparatus inlet (11a) and the check valve (13a) and is configured to convey respiratory gas in the direction of the check valve (13a) and the apparatus outlet (15a).

5. The respiratory apparatus (10) according to at least one of the preceding claims 1 to 4, **characterized in that** the bypass (17) branches off to the switching valve (14a) between the respiratory gas drive (12a) and the check valve (13a) and opens again into the respiratory gas path (16a) between the check valve (13a) and the apparatus outlet (15a).

6. The respiratory apparatus (10) according to one of claims 1 to 3, **characterized in that** the respiratory gas drive (12a) is arranged between the check valve (13a) and the apparatus outlet (15a) and is configured to convey respiratory gas in the direction of the apparatus outlet (15a).

7. The respiratory apparatus (10) according to claim 6, **characterized in that** the bypass (17) branches off to the switching valve (14a) between the apparatus inlet (11a) and the check valve (13a) and opens again into the inspiratory respiratory gas path (16a) between the check valve (13a) and the respiratory gas drive (12a).

8. The respiratory apparatus (10) according to at least one of the preceding claims, **characterized in that**, in addition to the bypass (17), a second inspiratory respiratory gas path (16b) is designed, which comprises a second apparatus inlet (11b) and at least one check valve (13b) and opens into the first inspiratory respiratory gas path (16a) upstream of the common apparatus outlet (15a).

9. The respiratory apparatus (10) according to at least one of the preceding claims, **characterized in that** a respiratory gas path (16c) for an expiratory respiratory gas flow is comprised which comprises an expiratory apparatus inlet (21) and an expiratory apparatus outlet (22a).

10. The respiratory apparatus (10) according to claim 9, **characterized in that**, from the expiratory apparatus inlet (21), the respiratory gas path (16c) for the expiratory respiratory gas flow leads to the expiratory apparatus outlet (22a) and a switching valve (14b) and a flow measuring device (18c) are arranged in the respiratory gas path (16c) for the expiratory respiratory gas flow.

11. The respiratory apparatus (10) according to claim 10, **characterized in that**, between the expiratory apparatus inlet (21) and the switching valve (14b), a separate expiratory respiratory gas path (16d) with a switching valve (14c) and a flow measuring device (18d) leads to a separate apparatus outlet (22b).

12. The respiratory apparatus (10) according to at least one of the preceding claims, **characterized in that** a tube system (19) that leads with a first branch (24) to a patient interface (20) and leads with a second branch (25) upstream of the patient interface (20) to the expiratory apparatus inlet (21) for expiratory respiratory gas is adapted at the apparatus outlet (15a).

13. The respiratory apparatus (10) according to at least one of claims 1-4 or 6, **characterized in that** the switching valve (14a) is arranged in the bypass (17), wherein the bypass (17) is designed as an open bypass which comprises a separate apparatus outlet (15b), wherein the open bypass (17) branches off from the inspiratory respiratory gas path (16a) between the check valve (13a) and the apparatus outlet (15a) and opens into the separate apparatus outlet (15b), wherein the inspiratory respiratory gas path (16a) has at least one further valve (26) and a flow measuring device (27), wherein the flow measuring device (27) is arranged between the respiratory gas drive (12a) and the check valve (13b).

14. The respiratory apparatus (10) according to at least one of the preceding claims 1-12, **characterized in that** the bypass is closed and opens into the respiratory gas path (16a) from which it branches off and thereby bypasses the check valve (13a).

15. The respiratory apparatus (10) according to claim 8, **characterized in that** the second inspiratory respiratory gas path (16b) extends from the second apparatus inlet (11b) to the common apparatus outlet (15a), wherein the second inspiratory respiratory gas path (16b) opens into inspiratory respiratory gas path (16a) between the check valve (13a) and the common apparatus outlet (15a), wherein, when the inspiratory respiratory gas path (16a) is blocked/disrupted, respiratory gas can be drawn via the second inspiratory respiratory gas path (16b) via the separate apparatus inlet (11b) and supplied to the patient, and additionally, when an expiratory respiratory gas path is blocked, respiratory gas can be fed back through the switching valve (14a), by bypassing the check valve (13a), and an exhalation can be enabled for the patient.

## Revendications

1. Appareil respiratoire (10) comportant au moins une voie de gaz respiratoire expiratoire, comprenant au moins une entrée d'appareil (11a) et une sortie d'appareil (15a) et une voie de gaz respiratoire inspiratoire (16a) entre l'entrée d'appareil (11a) et la sortie d'appareil (15a), dans lequel un entraînement de gaz respiratoire (12a), une soupape antiretour (13a) et une soupape de commande (14a) sont disposés dans la voie de gaz respiratoire inspiratoire (16a), dans lequel la soupape antiretour (13a) est disposée dans la voie de gaz respiratoire inspiratoire (16a) et configurée pour empêcher un flux de gaz respiratoire dans une direction allant de la sortie d'appareil (15a) vers l'entrée d'appareil (11a) et dans lequel la soupape de commande (14a) est configurée pour évacuer du gaz respiratoire du patient par le biais de la voie de gaz respiratoire inspiratoire (16a) en cas de blocage de la voie de gaz respiratoire expiratoire, par contournement de la soupape antiretour (13a) au moyen de la soupape de commande (14a), et pour permettre en revanche au moins partiellement un flux de gaz respiratoire dans une direction allant de la sortie d'appareil (15a) vers l'entrée d'appareil (11a) ou vers une ouverture d'appareil séparée, dans lequel la soupape de commande (14a) est disposée dans une dérivation (17) vers la soupape antiretour (13a), **caractérisé en ce qu'**un dispositif de mesure d'écoulement (18a) est disposé entre la soupape antiretour (13a) et la sortie d'appareil (15a), **en ce que** le dispositif de mesure d'écoulement (18a) est configuré pour détecter un volume du gaz respiratoire dans la voie de gaz respiratoire et **en ce que** la soupape de commande (14a) est configurée pour pouvoir être ouverte ou fermée sur la base du volume de gaz respiratoire détecté, permettant ainsi une commutation de la soupape de commande (14a) indépendamment du volume de gaz respiratoire.

2. Appareil respiratoire (10) selon la revendication 1, **caractérisé en ce que** l'au moins une soupape de commande (14a) forme une dérivation (17) autour de la soupape antiretour (13a).

3. Appareil respiratoire (10) selon l'une au moins des revendications précédentes, **caractérisé en ce que** l'au moins une soupape de commande (14a) est configurée pour ouvrir et/ou fermer la dérivation (17), dans lequel la soupape de commande (14a) est configurée de façon à pouvoir être commutée automatiquement.

4. Appareil respiratoire (10) selon l'une au moins des revendications précédentes, **caractérisé en ce que** l'entraînement de gaz respiratoire (12a) est disposé entre l'entrée d'appareil (11a) et la soupape antiretour (13a) et configuré pour transporter du gaz respiratoire en direction de la soupape antiretour (13a) et de la sortie d'appareil (15a).

5. Appareil respiratoire (10) selon l'une au moins des revendications précédentes 1 à 4, **caractérisé en ce que** la dérivation (17) vers la soupape de commande (14a) bifurque entre l'entraînement de gaz respiratoire (12a) et la soupape antiretour (13a) et débouche de nouveau dans la voie de gaz respiratoire (16a) entre la soupape antiretour (13a) et la sortie d'appareil (15a).

6. Appareil respiratoire (10) selon l'une des revendications 1 à 3, **caractérisé en ce que** l'entraînement de gaz respiratoire (12a) est disposé entre la soupape antiretour (13a) et la sortie d'appareil (15a) et configuré pour transporter du gaz respiratoire en direction de la sortie d'appareil (15a).

7. Appareil respiratoire (10) selon la revendication 6, **caractérisé en ce que** la dérivation (17) vers la soupape de commande (14a) bifurque entre l'entrée d'appareil (11a) et la soupape antiretour (13a) et débouche de nouveau dans la voie de gaz respiratoire inspiratoire (16a) entre la soupape antiretour (13a) et l'entraînement de gaz respiratoire (12a).

8. Appareil respiratoire (10) selon l'une au moins des revendications précédentes, **caractérisé en ce qu'**une deuxième voie de gaz respiratoire inspiratoire (16b) est réalisée en plus de la dérivation (17), laquelle comporte une deuxième entrée d'appareil (11b) et au moins une soupape antiretour (13b) et débouche dans la première voie de gaz respiratoire inspiratoire (16a) en amont de la sortie d'appareil (15a) commune.

9. Appareil respiratoire (10) selon l'une au moins des revendications précédentes, **caractérisé en ce qu'**il comporte une voie de gaz respiratoire (16c) destinée à un flux de gaz respiratoire expiratoire, laquelle comporte une entrée d'appareil expiratoire (21) et une sortie d'appareil expiratoire (22a).

10. Appareil respiratoire (10) selon la revendication 9, **caractérisé en ce qu'**à partir de l'entrée d'appareil expiratoire (21), la voie de gaz respiratoire (16c) destinée au flux de gaz respiratoire expiratoire mène vers la sortie d'appareil expiratoire (22a) et une soupape de commande (14b) ainsi qu'un dispositif de mesure d'écoulement (18c) sont disposés dans la voie de gaz respiratoire (16c) destinée au flux de gaz respiratoire expiratoire.

11. Appareil respiratoire (10) selon la revendication 10, **caractérisé en ce qu'**entre l'entrée d'appareil expiratoire (21) et la soupape de commande (14b), une voie de gaz respiratoire expiratoire séparée (16d) dotée d'une soupape de commande (14c) et d'un dispositif de mesure d'écoulement (18d) mène vers une sortie d'appareil séparée (22b).

12. Appareil respiratoire (10) selon l'une au moins des revendications précédentes, **caractérisé en ce qu'**un système de tubes (19) est adapté à la sortie d'appareil (15a), dont une première branche (24) mène vers une interface patient (20) et dont une deuxième branche (25) mène vers l'entrée d'appareil expiratoire (21) destinée à du gaz respiratoire expiratoire en amont de l'interface patient (20).

13. Appareil respiratoire (10) selon l'une au moins des revendications 1 à 4 ou 6, **caractérisé en ce que** la soupape de commande (14a) est disposée dans la dérivation (17), dans lequel la dérivation (17) est réalisée comme une dérivation ouverte comportant une sortie d'appareil séparée (15b), dans lequel la dérivation ouverte (17) bifurque à partir de la voie de gaz respiratoire inspiratoire (16a) entre la soupape antiretour (13a) et la sortie d'appareil (15a) et débouche dans la sortie d'appareil séparée (15b), dans lequel la voie de gaz respiratoire inspiratoire (16a) présente au moins une soupape supplémentaire (26) et un dispositif de mesure d'écoulement (27), dans lequel le dispositif de mesure d'écoulement (27) est disposé entre l'entraînement de gaz respiratoire (12a) et la soupape antiretour (13b).

14. Appareil respiratoire (10) selon l'une au moins des revendications 1 à 12, **caractérisé en ce que** la dérivation est fermée et débouche dans la voie de gaz respiratoire (16a) à partir de laquelle elle bifurque et contourne ainsi la soupape antiretour (13a).

15. Appareil respiratoire (10) selon la revendication 8, **caractérisé en ce que** la deuxième voie de gaz respiratoire inspiratoire (16b) s'étend à partir de la deuxième entrée d'appareil (11b) vers la sortie d'appareil (15a) commune, dans lequel la deuxième voie de gaz respiratoire inspiratoire (16b) débouche dans la voie de gaz respiratoire inspiratoire (16a) entre la soupape antiretour (13a) et la sortie d'appareil (15a) commune, dans lequel, en cas de blocage/panne de la voie de gaz respiratoire inspiratoire (16a), du gaz respiratoire peut être obtenu par le biais de l'entrée d'appareil séparée (11b) par le biais de la deuxième voie de gaz respiratoire inspiratoire (16b) et amené vers le patient et, en cas de blocage d'une voie de gaz respiratoire expiratoire, du gaz respiratoire peut en outre être renvoyé par la soupape de commande (14a) par contournement de la soupape antiretour (13a) et permettre au patient d'effectuer une exhalation.
